# EUROPEAN PATENT SPECIFICATION

(11) **EP 2 586 397 B1**
(45) Date of publication and mention of the grant of the patent: **17.10.2018**
(21) Application number: 12182887.5
(22) Date of filing: 01.05.2006
(51) Int. Cl.: A61B 50/20, A61B 50/30

(54) **Medical implement distribution and collection system**
System zur Verteilung und Sammlung medizinischer Instrumente
Système de distribution et de collecte d'instruments médicaux

(30) Priority: 02.05.2005 US 119960; 02.05.2005 US 119967; 02.05.2005 US 120119
(43) Date of publication of application: 01.05.2013
(62) Divisional of application: 06752051.0
(73) Proprietor: Covidien AG, 8212 Neuhausen am Rheinfall (CH)
(72) Inventor: Smudde, Anton M., Elk Grove Village, IL 60007 (US); Brown, Robert A., Algonquin, IL 60102 (US)
(74) Representative: Prock, Thomas

(56) References cited:
- DE-C- 538 682
- US-A- 5 143 210
- US-A- 5 152 394
- US-A- 5 251 783
- US-A- 5 706 942
- US-A1- 2003 132 129
- US-B2- 6 702 147

## Description

### FIELD OF THE INVENTION

The present invention relates to a distribution and collection system for medical implements.

### BACKGROUND OF THE INVENTION

In a hospital, doctor's office or home environment setting, soiled syringes or other medical implements are commonly deposited In a disposal container following their use. Unused, sterile syringes or other medical implements are commonly obtained from a source separate from the disposal container. It is somewhat inconvenient at times, however, to provide a source of unused, sterile medical implements and a separate container for collecting the soiled medical implements following their use. In other words, it is sometimes disadvantageous from a convenience standpoint for medical practitioners to have one location from which implements are obtained and a separate location in which implements are disposed of.

Attempts have been made to overcome this inconvenience. For example, improved syringe dispensing and collecting systems for personal use are disclosed in U.S. Patent No. 5,152,394 , which illustrates a syringe dispensing and collecting system comprising a cylindrical container having a sterile hypodermic needle storage chamber and a separate soiled hypodermic needle collection chamber. The storage chamber is maintained in an outer peripheral region of the cylindrical container and the collection chamber is maintained in a central region of the cylindrical container. The storage chamber and the collection chamber are separated by an inner cylindrical wall. A telescoping cover mounted to the top of the container defines an opening configured to accept a soiled hypodermic needle. An outer wall of the container provides an outlet opening for the passage of sterile hypodermic needles from the storage chamber.

Several other references disclose medical waste disposal and/or dispensing apparatus. U.S. Publication 2003/0132129 discloses a container having a dividing tray to provide a movable barrier between used syringes and unused syringes. German Patent DE 538 682 discloses a device for storing and dispensing cotton balls including containers for dispensing cotton balls and a waste container for receiving cotton balls attached to a fixed base. U.S. Patent 5,143,210 discloses a carton for displaying and dispensing chemical reagents for use in performing diagnostic tests. U.S. Patent 5,251,783 discloses a utility blade housing and carrier for dispensing and disposing of utility blades. U.S. Patent 5,152,394 discloses a syringe dispensing and disposal container having an inner collection chamber for used syringes and an outer storage chamber for unused syringes. U.S. Patent 6,702,147 discloses a housing having apertures in a top surface thereof for dispensing and disposing medicated wipes. The housing is configured to receive a dispensing container and a disposal container for dispensing and disposing the wipes. U.S. Patent 5,706,942 discloses a surgical blade dispenser and disposal apparatus including a blade removal structure for disposing surgical blades and a blade support structure for supporting a plurality of unused surgical blades.

Nevertheless, there continues to be a need to further develop and improve disposal and collection devices for medical implements.

### SUMMARY OF THE INVENTION

The present invention provides a medical implement dispensing and disposal system as defined in appended claim 1. Preferred embodiments are defined in the dependent claims.

According to an aspect of the invention a medical implement dispensing and disposal system is configured for mounting within an interior of an enclosure having a first opening for receiving soiled medical implements and a second opening for dispensing medical implements. The medical implement dispensing and disposal system comprises a dispensing chamber configured to be substantially enclosed within the interior of the enclosure and removed from the interior of the enclosure. The dispensing chamber is configured to contain medical implements and has an access opening for passage of medical implements from the dispensing chamber. The access opening is positioned for alignment with the second opening of the enclosure to facilitate passage of medical implements from the enclosure. The medical implement dispensing and disposal system further comprises a disposal chamber configured to be substantially enclosed within the interior of the enclosure adjacent the dispensing chamber and removed from the interior of the enclosure. The disposal chamber is further configured to collect soiled medical implements and has an inlet opening for passage of soiled medical implements into the disposal chamber. The inlet opening is positioned for alignment with the first opening of the enclosure to facilitate passage of soiled medical implements into the enclosure.

According to another aspect of the invention, a medical implement dispensing and disposal system comprises an enclosure having an interior. A disposal chamber is positioned within the interior of the enclosure and defines an inlet opening for passage of soiled medical implements into the disposal chamber. A dispensing chamber is positioned within the interior of the enclosure and defines an access opening for passage of medical implements from the dispensing chamber.

According to yet another aspect of the invention, an enclosure configured to accommodate a medical implement dispensing and disposal system having a dispensing chamber for dispensing medical implements and a disposal chamber for receiving soiled medical implements is provided. The enclosure comprises an enclosure body defining an interior configured to receive the medical implement dispensing and disposal system. An inlet opening is defined by the enclosure body, wherein the inlet opening is positioned for passage of soiled medical implements from outside the enclosure body Into the disposal chamber within the interior of the enclosure body. An access opening is defined by the enclosure body, wherein the access opening is positioned for passage of medical Implements from within the dispensing chamber within the interior of the enclosure body.

Another aspect of the invention provides a container configured to dispense and dispose medical Implements comprising a body portion, a hood portion extending from the body portion and an access opening formed at least partially In the hood portion. In a first orientation, the container is configured to collect soiled medical implements through the access opening of the hood portion and the body portion of the container is configured to contain soiled medical implements. In a second orientation, the container is configured to dispense medical implements through the access opening of the hood portion. The body portion of the container Is configured to contain the medical implements, the hood portion is oriented to limit the unintentional escapement of medical implements from the body portion, and the access opening is positioned for passage of medical implements from the body portion. The second orientation of the container is inverted with respect to the first orientation of the container.

### BRIEF DESCRIPTION OF THE DRAWINGS

The invention is best understood from the following detailed description when read in connection with the accompanying drawings. It is emphasized that, according to common practice, the various features of the drawings are not to scale. On the contrary, the dimensions of the various features are arbitrarily expanded or reduced for clarity. Included in the drawings are the following figures:
FIG. 1 is a perspective view of an embodiment of a medical implement dispensing and disposal system according to an aspect of this invention;
FIG. 2 is a perspective view of the embodiment illustrated in FIG. 1 mounted within a closed enclosure;
FIG. 3 is a perspective view of the embodiment Illustrated in FIG. 1 mounted within an open enclosure;
FIG. 4 is a perspective view of another embodiment of a medical Implement dispensing and disposal system according to an aspect of this invention;
FIG. 5 is a perspective view of the embodiment illustrated in FIG. 4 mounted within a closed enclosure;
FIG. 6 is a perspective view of the embodiment illustrated In FIG. 4 mounted within an open enclosure;
FIG. 7 is a perspective view of yet another embodiment of a medical implement dispensing and disposal system according to an aspect of this invention;
FIG. 7A is a perspective view of the embodiment illustrated in FIG. 7 in conjunction with a flip lid;
FIG. 8 is a perspective view of the embodiment illustrated in FIG. 7 mounted within a closed enclosure; and
FIG. 9 is a perspective view of the embodiment illustrated in FIG. 7 mounted within an open enclosure.

### DETAILED DESCRIPTION OF THE INVENTION

Although the invention is illustrated and described herein with reference to specific embodiments, the invention is not intended to be limited to the details shown. Rather, various modifications may be made in the details of the invention.

The invention is best understood from the following detailed description when read in connection with the accompanying drawing figures, which shows exemplary embodiments of the invention selected for illustrative purposes. The invention will be illustrated with reference to the figures. Such figures are intended to be illustrative rather than limiting and are included herewith to facilitate the explanation of the present invention.

As used herein, the term medical implement refers to any commonly consumed device used for medical purposes, such as but not limited to a sharp, syringe, tongue depressor, lancet, scalpel, slide, pipette and the like.

Referring generally to the figures, a medical implement dispensing and disposal system 100, 200, 300 according to exemplary aspects of this invention is comprised of a medical implement dispensing container or container portion 120, 220, 310' defining a dispensing chamber; a soiled medical implement collection container or container portion 110, 210, 310 defining a collection chamber; and an optional enclosure 130, 230, 330 to house both chambers. The collection chamber defined by container 110, 210, 310 is configured to collect soiled medical implements and includes an inlet 111, 211, 317 positioned for the passage of the soiled medical implements into the collection container 110, 210, 310. The dispensing chamber defined by container 120, 220, 310' is configured to contain unused medical implements and includes an access opening 129, 223, 317 positioned for the passage of unused medical implements into the container 120, 220, 310'.

The dispensing and collection chambers are housed within the enclosure 130, 230, 330. The enclosure 130, 230, 330 provides an opening 131, 231, 331 to access the inlet 111, 211, 317 of the collection chamber defined by container 110, 210, 310. The enclosure 130, 230, 330 also provides an opening 132, 232, 332 to access the access opening 129, 223, 317 of the dispensing chamber defined by container 120, 220, 310'. The enclosure 130, 230, 330 includes a locking door 135, 235, 335 to inhibit unauthorized access to the dispensing container 120, 220, 310' and the collection container 110, 210, 310.

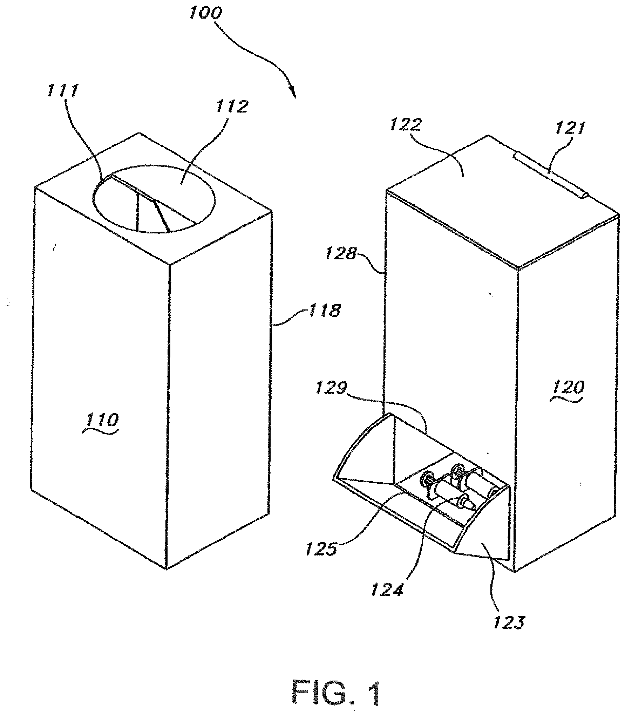

Although an enclosure 130, 230, 330 is selected for illustration, an enclosure 130, 230, 330 is an optional component of the medical implement dispensing and disposal system 100, 200, 300. A medical implement dispensing and disposal system 100, 200, 300 may include only a dispensing chamber such as the one defined by the dispensing container 120, 220, 310' and a collection chamber such as the one defined by the collection container 110, 210, 310, as illustrated in Figures 1, 4 and 7.

Referring to the exemplary embodiment illustrated in Figures 1 through 3, more specifically Figure 1, a medical Implement dispensing and disposal system according to one aspect of this invention is generally designated by the numeral 100. The system 100 includes a collection chamber defined by a collection container 110 and a dispensing chamber defined by a dispensing container 120. The collection container 110 includes a body portion 118, a lid 112 and an inlet 111 formed in the lid 112. The dispensing container 120 includes a body portion 128, a lid 122, a chute 123 and an access opening 129.

The collection container 110 of the exemplary embodiment is configured to store soiled medical implements. The walls of the collection container 110 define an interior body portion 118 and compose the structure of the collection container 110. The soiled medical implements are stored within the interior body portion 118. A lid 112 is positioned on the top wall of the collection container 110 to provide access to the body portion 118. The lid 112 defines an inlet opening 111 for passage of the soiled medical implements into the body portion 118.

The lid 112 comprises all or a portion of the top wall of the collection container 110. In this embodiment, the lid 112 includes a slideable screen that is pivotable between an open position (as illustrated in Figure 1) and a closed position. The position of the screen defines the size of the inlet opening 111 through which the soiled medical implements are passed. The user pivots the screen to close the inlet opening 111, for example, to restrict access to the interior body portion 118 filled with soiled medical implements. The user may also close the inlet opening 111 to prevent the leakage of soiled medical implement or contents thereof during transportation.

The lid 112 may be integrally formed with the top wall of the collection container. Alternatively, the lid 112 may be a discrete component mounted to the top wall of the collection container as shown in Figure 1. The lid 112 can be mounted to the top wall of the collection container 110 using a hinge, adhesive, weld, clip, damp or any other mechanical fastening method commonly known in the art. Although the lid 112 and integral inlet 111 are positioned on the top wall of the collection container 110 in this embodiment, the lid 112 and inlet 111 could be positioned on the upper portion of any side wall of the collection container 110 or at any other location, depending on other aspects of the design.

The dispensing container 120 of the exemplary embodiment is configured to dispense sterile, unused medical Implements 124. The walls of the dispensing container 120 define the interior body portion 128 and compose the structure of the dispensing container 120. The sterile medical implements 124 are stored within the interior body portion 128. A lid 122 is hingedly connected to the top end of the dispensing container 120 by a hinge 121 to provide access to the interior body portion 128. The lid 122 pivots between an open position and a closed position (as illustrated in Figure 1). In use, the lid 122 is pivoted to an open position to load sterile medical implements 124 into the interior body portion 128 of the dispensing container 120. The lid 122 is pivoted to a closed position, as shown, to inhibit access to the sterile medical implements 124.

An access opening 129 is formed on the lower end of a side wall to provide access to the sterile medical implements 124. An extendable chute 123 is hingedly connected to the bottom end of the dispensing container 120 by a hinge 125. The chute 123 comprises two side walls and a front wall extending between the side walls. The extendable chute 123 pivots between an open position (as illustrated in Figure 1) and a closed position. In the open position, the extendable chute 123 facilitates the controlled passage of sterile medical implements 124 from the body portion 128 via access opening 129. In the open position, the chute 123 also forms an effective barrier to prevent the medical implements 124 from uncontrollably surging out of the access opening 129. In the closed position, the chute 123 obstructs the access opening 129, thereby preventing access to the sterile medical implements 124 within the body portion 128.

For the purposes of shipping and handling, the chute 123 and lid 122 are maintained in the closed position to prevent the escapement of pre-packaged sterile medical implements 124. The chute 123 and lid 122 are also maintained in the closed position to reduce the overall shipping size of the dispensing container 120. Although not illustrated, the chute 123 and lid 122 may incorporate locking features, to further prevent unauthorized access to the sterile medical implements 124.

In use and according to the exemplary embodiment illustrated in Figure 1, one or more of the pre-packaged sterile medical implement(s) 124 are removed from the extended chute 123 of the dispensing container 120 for use, i.e. soiling. The formerly sterile medical implement(s), now soiled, are prepared for disposal and then inserted into the body portion 118 via inlet opening 111. After the body portion 118 of the collection container 110 is filled to capacity, the lid 112 is closed to prevent access to the body portion 118 through the inlet opening 111.

The containers 110, 120 of the exemplary embodiment may be formed by an injection molding process or blow molding process or any known forming process. Alternatively, the walls of the containers may be separate and adhered, welded, snapped and/or clipped together. The containers 110, 120 are desirably composed of a substantially leak resistant material such as polypropylene or polyethylene. The containers 110, 120 may be partially or completely transparent or translucent for the purpose of monitoring the level of medical implements within the containers.

Although not illustrated, a single universal container could incorporate the features of both containers 110 and 120. The universal container would provide both a lid 112 (with inlet opening 111) and an extendable chute 123. When used as a dispensing container, the extendable chute 123 of the universal container would be extended to an open position and the lid 112 would be rotated to a closed position. When used as a collection container, the extendable chute 123 of the universal container would be retracted to a closed position and the lid 112 would be rotated to an open position. Manufacturing, inventory and/or tooling a single universal dispensing/collection container in lieu of two separate containers could represent a significant cost savings.

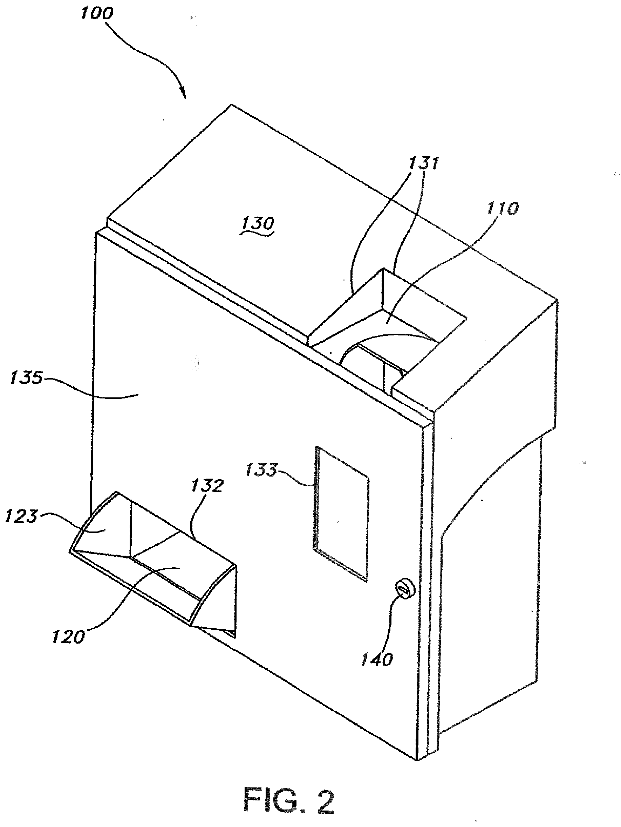

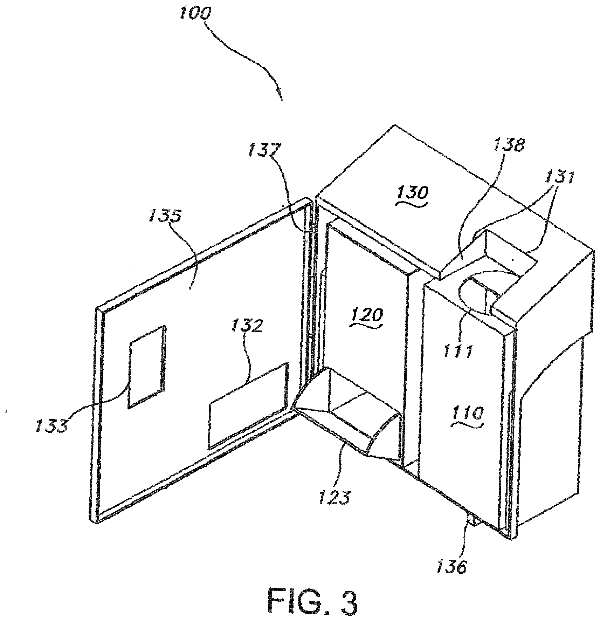

Referring specifically now to Figures 2 and 3, the medical implement dispensing and disposal system 100 illustrated in Figure 1 is mounted in an enclosure 130, according to one aspect of this invention. As mentioned previously, although an enclosure 130 is selected for illustration and included as a component of this exemplary embodiment, the enclosure 130 is an optional component of the dispensing and disposal system 100.

Figure 2 illustrates the medical implement dispensing and disposal system 100 maintained in an enclosure 130, wherein the door 135 of the enclosure 130 is in a closed position. Figure 3 illustrates the door 135 of the enclosure 130 in an open position. The enclosure 130 accommodates the containers 110, 120 for storage and safety purposes without inhibiting the functionality of the collection container 110 and the dispensing container 120. Accordingly, the enclosure 130 provides an opening 132 to accommodate the extendable chute 123 of the dispensing container 120. The opening 132 provides adequate clearance so that the extendable chute 123 may extend through the enclosure door 135 without obstruction (as illustrated in Figure 2).

The enclosure 130 also provides an inlet opening 131 substantially aligned with the inlet opening 111 of the collection container 110 to permit the passage of soiled medical implements through the inlet opening 111. The inlet opening 131 includes a plurality of side walls 138 that extend into or toward the interior of the enclosure 130 to contact or terminate proximal the top side of the collection container 110. The side walls 138 prohibit the soiled medical implements from unintentionally descending Into the interior of the enclosure 130 or entering the chute 123.

The enclosure 130 comprises five sidewalls and a hingedly connected door 135. Although the enclosure 130 comprises five sidewalls, the enclosure may have any number of sidewalls. The door 135 is hingedly connected to a sidewall of the enclosure 130, by a hinge 137, as illustrated in Figure 3. The door 135 may be connected to any of the sidewalls of the enclosure 130, as the orientation and position of the door 135 is not limited to the illustration shown.

One or more supports 136 are provided on the base of the enclosure 130. The supports 136 are configured to maintain the enclosure 130 in an upright position when the enclosure 130 is mounted on a surface such as a floor or desk. Alternatively, although not shown, the rear wall of the enclosure 130 may provide holes, slots or brackets for mounting the enclosure 130 to a wall.

A window 133 is provided on the door 135 of the enclosure 130. The window facilitates the monitoring of soiled medical implements within the collection container 110 when the door 135 is in the closed position, as illustrated in Figure 2. In use, a user monitors the level of the soiled medical implements within the collection container 110 to determine when to replace the collection container 110.

A lock 140 is provided on the door 135 of the enclosure 130 to prevent unauthorized access to the interior of the enclosure 130. The lock 140 engages with a side wall of the enclosure 130.

The enclosure 130 of the exemplary embodiment may be formed by an injection molding, blow molding, casting or other forming process. Alternatively, the walls of the enclosure may be separate and adhered, welded, snapped and/or clipped together. The enclosure 130 may be composed of a material such as polypropylene or polyethylene or other suitable material.

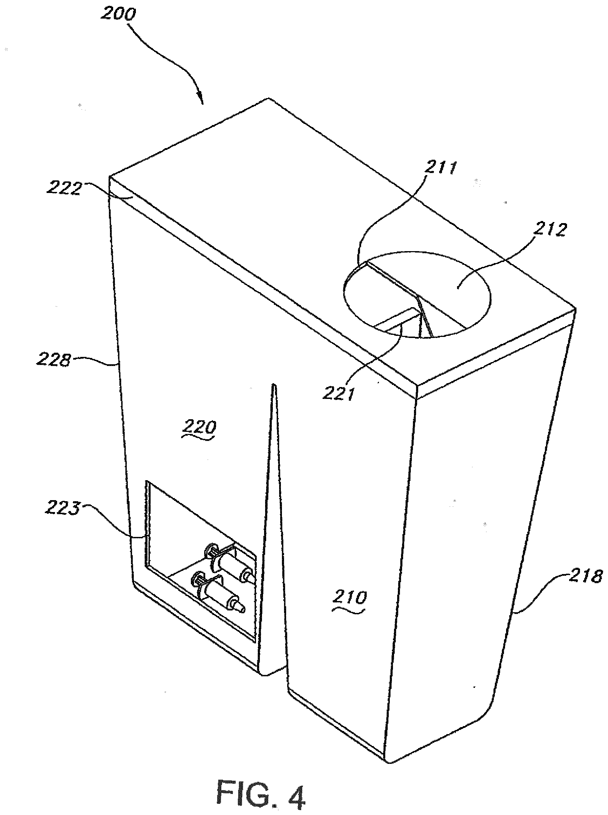

Similar to the exemplary embodiment illustrated in Figures 1 through 3, another exemplary embodiment of a medical implement dispensing and disposal system is illustrated in Figures 4 through 6. The medical implement dispensing and disposal system 200 includes a collection container 210 defining a collection chamber configured to collect soiled medical implements and a dispensing container 220 defining a dispensing chamber configured to dispense medical implements. In this exemplary embodiment the dispensing container 220 and the collection container 210 are formed from a single unitized body. Manufacturing, inventory and/or tooling of a single dispensing/collection container in lieu of two separate containers could represent a significant cost savings. The dispensing container 220 includes a body portion 228 and an access opening 223. The collection container 210 includes a body portion 218. A lid 222 is mounted to the top end of the collection container 210 and the dispensing container 220.

Similar to the previous embodiment illustrated In Figures 1 through 3, the lid 222 incorporates a slideable screen 212 that is pivotable between an open position (as illustrated in Figure 4) and a closed position. The position of the screen 212 defines the size of the inlet opening 211 through which the soiled medical implements are passed. The slideable screen 212 may be integral with the lid 222 or mounted (e.g. snapped or adhered) onto the lid 222 as illustrated in Figure 4.

The collection container 210 and the dispensing container 220 are formed from a single unitized body. An interior wall 221, shown as a dotted line, is positioned within the interior of the containers and separates the collection container 210 from the dispensing container 220. The interior wall 221 prevents the integration of the soiled and sterile medical implements. The access opening 223 accommodates the passage of sterile medical implements from the dispensing container 220. A lid 222 is provided to cover the exposed top side of the containers 210, 220. The lid 222 is mounted to the top side of the containers 210, 220 by any mechanical mounting means known in the art, e.g. tongue and groove, clips, clamps, welds, adhesive, etc.

The unitized containers 210, 220 of the exemplary embodiment may be formed by an injection molding process or blow molding process or other known manufacturing process. Alternatively, the walls of the containers may be separate and adhered, welded, snapped and/or clipped together. The containers 210, 220 are desirably composed of a substantially leak resistant material such as polypropylene or polyethylene. The containers 210, 220 may be partially or completely transparent or translucent for the purpose of monitoring the level of medical implements within the containers.

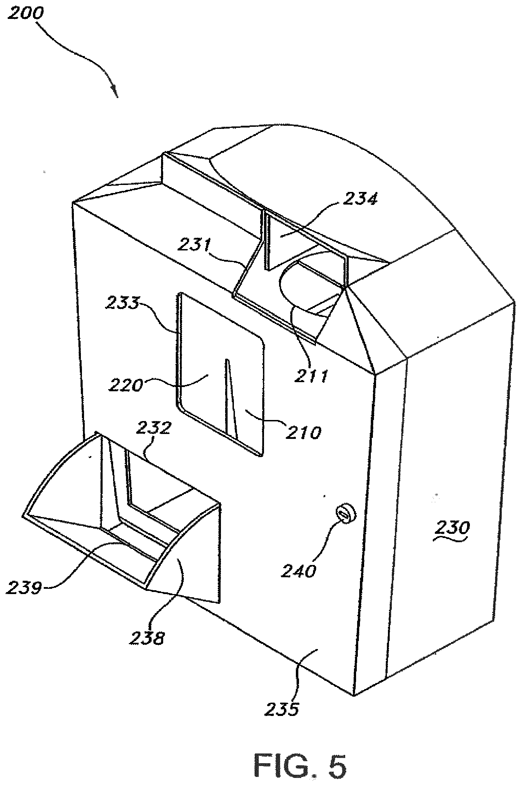

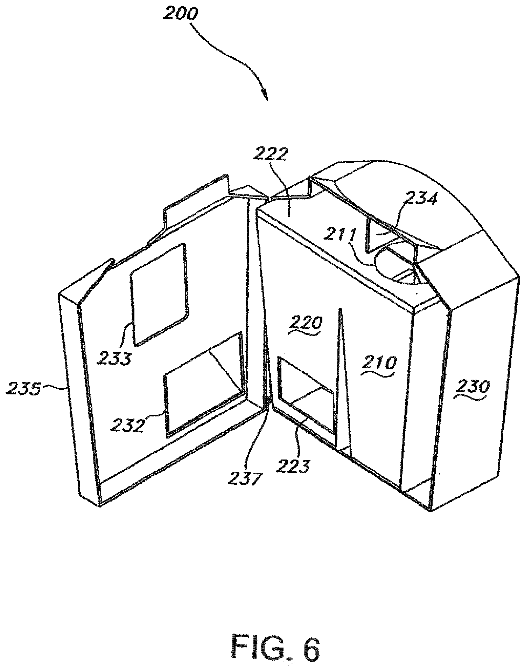

Referring specifically now to Figures 5 and 6, similar to the previous embodiment, the medical Implement dispensing and disposal system 200 illustrated in Figures 5 and 6 is mounted in an enclosure 230. Figure 5 illustrates the medical implement dispensing and disposal system 200 maintained in an enclosure 230, wherein the door 235 of the enclosure 230 is in a closed position. Figure 6 illustrates the door 235 of the enclosure 230 in an open position. The door 235 is hingedly connected to the enclosure 230 by a hinge 237. A window 233 is provided on the door 235 for monitoring the soiled medical implements within the collection container 210. A lock 240 is also provided on the door 235 to prevent unauthorized access to the Interior portion of the enclosure 230.

Unlike the previous embodiment however, the enclosure 230 of this exemplary embodiment incorporates the extendable chute 238. The chute 238 is hingedly connected to the door 235 by hinge 239 and aligned with the access opening 223 of the dispensing container 220. The extendable chute 238 facilitates the controlled passage of sterile medical implements 224 through an access opening 232 of the enclosure 230 and the access opening 223 of the dispensing container 220. In the open position, the chute 238 also forms an effective barrier to prevent the medical implements 224 from uncontrollably surging out of the access opening 223 of the dispensing container 220, while concurrently providing user access to the sterile medical implements 124. In the closed position, the chute 238 obstructs the access opening 223 of the dispensing container 220, thereby prohibiting unauthorized user access to the sterile medical implements 224. In the retracted position (i.e. closed position), the side walls of the chute 238 are positioned on either side of the dispensing container 220.

The enclosure 230 provides an opening 231 substantially aligned with the inlet opening 211 of the collection container 210 to permit the passage of soiled medical implements through the inlet opening 211. The inlet 231 of the enclosure 230 includes a plurality of side walls 234 extending Into the interior of the enclosure 230. The side walls 234 are maintained in frictional contact with the lid 222 and provide a barrier to prohibit the soiled medical implements from unintentionally descending into the interior of the enclosure 230.

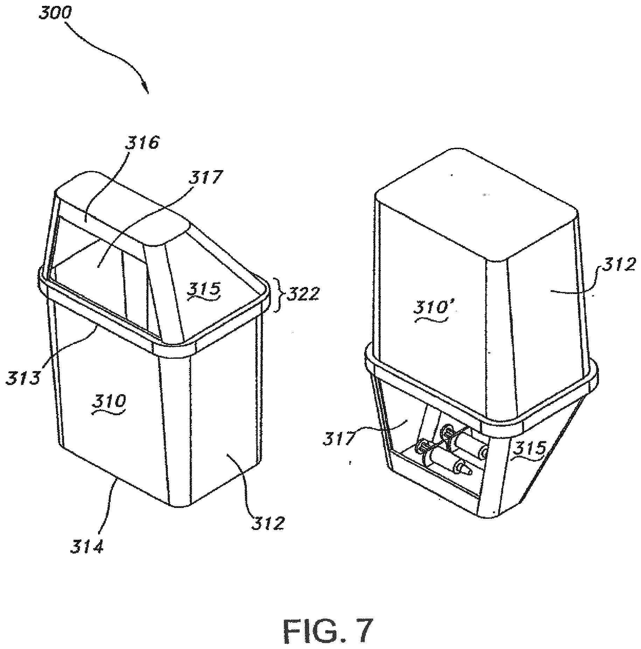

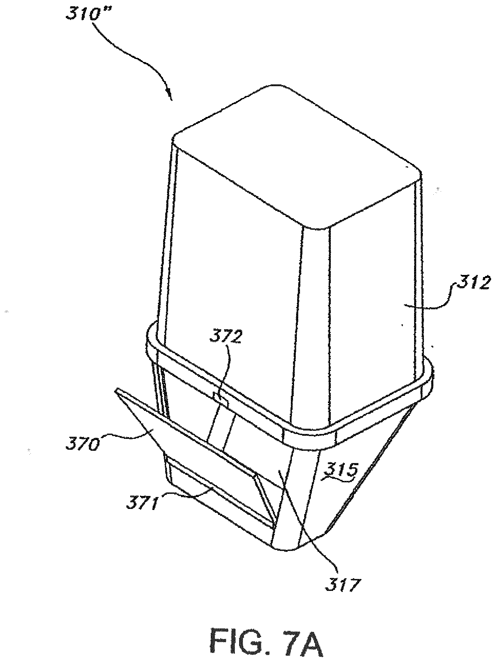

Similar to the exemplary embodiments illustrated In Figures 4 through 6, another exemplary embodiment of a medical implement dispensing and disposal system is illustrated in Figures 7 through 9. The medical implement dispensing and disposal system 300 includes a collection container 310 defining a collection chamber configured to collect soiled medical implements and a dispensing container 310' defining a dispensing chamber configured to facilitate the distribution of medical implements. In this exemplary embodiment a single universal container is configured to be both a dispensing container 310' and a collection container 310. In one orientation the container operates as a dispensing container 310' and in another orientation the container operates as a collection container 310. Referring specifically to Figure 7, the container shown to the left is oriented as a collection container 310 and the container shown to the right is oriented as a dispensing container 310'. The collection container 310 is oriented upright and the dispensing container 310' is oriented inverted.

The universal container 310, 310' may be advantageous from a manufacturing, inventory and/or tooling perspective. The fabrication of a single dispensing/collection container in lieu of two different containers may represent a significant cost savings.

The container 310, 310' Includes a body portion 312 and a lid 315. The lid 315 is removably mounted to the top side 313 of the body portion 312. The lid 315 may be integrated with the body portion 312 or a separate component as illustrated in Figure 7. The lid 315 includes an Integral flange portion 322 positioned along the periphery of the lid 315. The purposed of the flange portion 322 will be described in further detail later. The lid 315 may be composed of sheet-metal or formed by a molding process.

A barrier wall 316 formed in the lid 315 extends into an opening 317. In the dispensing orientation (310'), the barrier wall 316 contains the sterile medical implements to facilitate the controlled passage of sterile medical implements through the opening 317 of the lid 315. The barrier wall 316 also forms an effective barrier to prevent the medical implements from uncontrollably surging out of the opening 317.

The exemplary embodiment of the dispensing container 310" illustrated in Figure 7A includes a flip lid 370 pivotably coupled to the barrier wail 316. The flip lid 370 is configured to pivot between an open position and a closed position. The flip lid 370 illustrated in Figure 7A is shown in a partially open position. Although the flip lid 370 selected for illustration is incorporated with a dispensing container 310", the flip lid 370 may also be incorporated with a collection container 310. In the open position the flip lid 320 either provides access to the sterile medical Implements within the dispensing container 310" or the soiled medical implements within the collection container 310. In the closed position the flip lid 370 obstructs the opening 317 thereby prohibiting access to the soiled medical implements within the collection container 310 or the sterile medical implements within the dispensing container 310". A lock 372 is coupled to the barrier wall 316 to prevent unauthorized access to the containers 310, 310". The lock is especially advantageous to safely obstruct the opening 317 of the collection container 310 upon handling and transportation of the container 310.

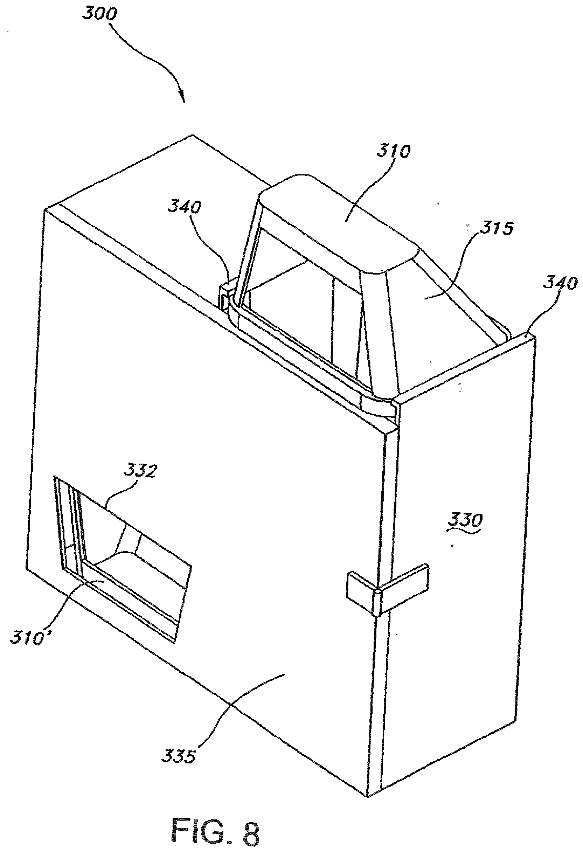

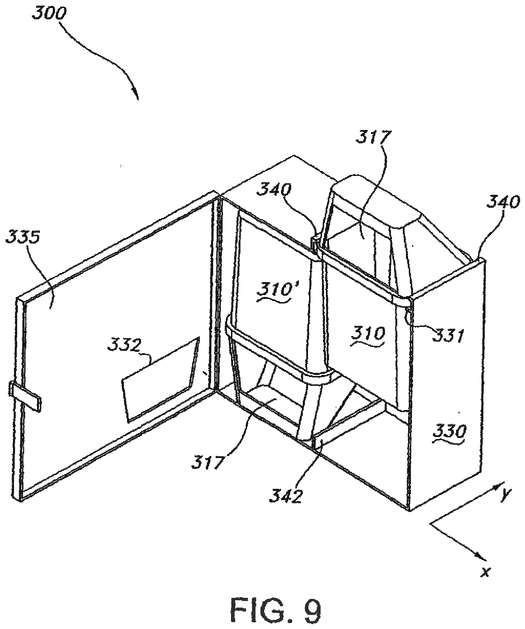

Referring specifically now to Figures 8 and 9, similar to the previous embodiments the medical implement dispensing and disposal system 300 illustrated in Figures 8 and 9 are accommodated in an enclosure 330. Figure 8 Illustrates the medical implement dispensing and disposal system 300 maintained in an enclosure 330, wherein the door 335 of the enclosure 330 is In a closed position. Figure 9 illustrates the door 335 of the enclosure 330 in an open position. As shown in Figure 9, the dispensing container 310' Is Illustrated on the left hand side of the enclosure 330 in an Inverted orientation and the collection container 310 is illustrated on the right hand side of the enclosure 330 in an upright orientation.

The enclosure 330 provides an opening 331 positioned to accommodate the lid 315 of the collection container 310. The enclosure 330 also provides an access opening 332 substantially aligned with the opening 317 of the dispensing container 310'to facilitate the passage of sterile medical implements from the dispensing container 310'. Two rail sections 340 accommodate the flange portions 322 of the collection container 310. The flange portions 322 engage with and translate along the rail sections 340. The two rail sections 340 limit the collection container 310 from shifting in the x direction. The door 335 and rear wall of the enclosure limit the collection container 310 from shifting in the y direction. A barrier 342 formed on the lower wall of the enclosure 330 limits the dispensing container 310' from shifting in the x direction. The door 335 and rear wall of the enclosure limits the dispensing container 310' from shifting in the y direction.

In use, after all of the sterile medical implements within the dispensing container 310' have been utilized and the collection container 310 is sufficiently filled with soiled medical implements, the filled collection container 310 is removed from the enclosure 330 and safely disposed of to provide space for an empty collection container 310. The empty dispensing container 310' is inverted to change its functionality from a dispensing container 310'to a collection container 310. The collection container 310, which was previously an empty dispensing container 310', is mounted on the right hand side of the enclosure 330 in an upright position. A new dispensing container 310' filled with sterile medical implements is mounted on the left hand side of the enclosure 330.

Referring to the embodiment illustrated in Figures 7 through 9, the sterile medical implements may be pre-packaged within the dispensing container 310' to facilitate quick installation of the system 300. The opening 317 may be sealed with a removable barrier to prevent the escapement of the sterile medical implements from the dispensing container 310' during shipment. It is contemplated that an enclosure 330 with or without a dispensing container 310' and/or a collection container 310 could be packaged and shipped to a user. It is also contemplated that an individual dispensing container 310' and/or a collection container 310 could be packaged and shipped to a user, with or without an enclosure 330.

Although this invention has been described with reference to particular embodiments selected for illustration in the Figures, it will be appreciated that many variations and modifications can be made to the systems 100, 200, 300 and the components thereof. For example, it should be noted that it is not required that the unused medical implements are sterile, as the dispensing container is configured to hold a medical implement in any condition. Although several molding processes are mentioned, the systems and components thereof are not limited to any specific manufacturing process or material.

## Claims

1. A medical implement dispensing and disposal system (100, 200, 300) comprising:
an enclosure (130, 230, 330) defining an interior and having a first opening (131, 231, 331) for receiving soiled medical implements and a second opening (132, 232, 332) for dispensing medical implements;
a dispensing chamber (120, 220, 310') configured to be substantially enclosed within the interior of the enclosure (130, 230, 330) and removed from the interior of the enclosure (130, 230, 330), said dispensing chamber (120, 220, 310') being adapted to contain medical implements and having an access opening (129, 223, 317) at a lower end of the dispensing chamber (120, 220, 310') for passage of medical implements from said dispensing chamber (120, 220, 310'), said access opening (129, 223, 317) being positioned for alignment with the second opening (132, 232, 332) of the enclosure (130, 230, 330) to facilitate passage of medical implements from the enclosure (130, 230, 330); and
a disposal chamber (110, 210, 310) configured to be substantially within the interior of the enclosure (130, 230, 330) adjacent said dispensing chamber (120, 220, 310') and removed from the interior of the enclosure (130, 230, 330), said disposal chamber (110, 210, 310) being adapted to collect soiled medical implements, said disposal chamber (110, 210, 310) having an inlet opening (111, 211, 317) at an upper end of the disposal chamber (110, 210, 310) for passage of soiled medical implements into said disposal chamber (110, 210, 310), said inlet opening (111, 211, 317) being positioned for alignment with the first opening (131, 231, 331) of the enclosure (130, 230, 330) to facilitate passage of soiled medical implements into the enclosure (130, 230, 330).

2. The medical implement dispensing and disposal system (200) of claim 1, said dispensing chamber (220) and said disposal chamber (210) being formed from a single unitized body.

3. The medical implement dispensing and disposal system (200) of claim 1, further comprising an interior wall portion (221) separating said dispensing chamber (220) and said disposal chamber (210).

4. The medical implement dispensing and disposal system (200) of claim 2, further comprising a lid (222) covering said dispensing chamber (220) and said disposal chamber (210).

5. The medical implement dispensing and disposal system (200) of claim 4, said lid including a door (212) positioned proximal said inlet opening (211) of said disposal chamber (210), said door (212) having an open position configured to permit access to an interior of said disposal chamber (210) and a closed position configured to inhibit access to the interior of said disposal chamber (210).

6. The medical implement dispensing and disposal system (200) of claim 5, said door (212) being mounted for rotation between said open and closed positions.

7. The medical implement dispensing and disposal system (100, 300) of claim 1, wherein said dispensing chamber (120, 310') and said disposal chamber (110, 310) are defined by separate containers.

8. The medical implement dispensing and disposal system (100, 300) of claim 7, said dispensing chamber (120, 310') including an inlet opening (129, 317) and a lid (122, 315) movable to an open position in which medical implements can be deposited into said dispensing chamber (120, 310') through said inlet opening (129, 317).

9. The medical implement dispensing and disposal system (100, 300) of claim 8, said lid (122, 315) being hingedly connected adjacent said inlet opening (129, 317), said lid (122, 315) being configured to pivot between said open position and a closed position.

10. The medical implement dispensing and disposal system (100) of claim 7, said dispensing chamber (120) including a trough portion (123) positioned proximal a base of said dispensing chamber (120), said trough portion (123) at least partially defining said access opening (129).

11. The medical implement dispensing and disposal system (100) of claim 10, wherein said trough portion (123) comprises a door movable between an open position and a closed position, and where said trough portion (123) provides a passage through said access opening (129) when said door is in said open position and said trough portion (123) at least partially obstructs said access opening (129) when said door is in said closed position.

12. The medical implement dispensing and disposal system (100) of claim 11, wherein said door is pivotable between said open position and said closed position.

13. The medical implement dispensing and disposal system (100) of claim 11, said door having a wall at least partially forming said trough portion (123) when in said open position.

14. The medical implement dispensing and disposal system (100) of claim 13, said door having side wall portions and a front wall portion extending between said side wall portions.

15. The medical implement dispensing and disposal system (100, 200, 300) of claim 1, said dispensing chamber (120, 220, 310') containing at least one medical implement.

16. The medical implement dispensing and disposal system (100, 200, 300) of claim 15, wherein said at least one medical implement is selected from the group consisting of syringes, sharps, tongue depressors or swabs.

17. The medical implement dispensing and disposal system (100, 300) of claim 7, said disposal chamber (110, 310) including a door (112, 370) positioned proximal said inlet opening (111, 317), said door (112, 370) having an open position configured to permit access to an interior of said disposal chamber (110, 310) and a closed position configured to inhibit access to the interior of said disposal chamber (110, 310).

18. The medical implement dispensing and disposal system (100, 300) of claim 17, said door (112, 370) being mounted for rotation between said open and closed positions.

19. The medical implement dispensing and disposal system (300) of claim 1, wherein said dispensing chamber (310') and said disposal chamber (310) are each defined by substantially identical containers (310, 310').

20. The medical implement dispensing and disposal system (300) of claim 19, said containers (310, 310') defining said dispensing chamber (310') and said disposal chamber (310) each having a lid (315).

## Patentansprüche

1. System zum Spenden und Entsorgen (100, 200, 300) medizinischer Geräte, Folgendes beinhaltend:
eine Einhausung (130, 230, 330), welche ein Inneres definiert und eine erste Öffnung (131, 231, 331) zur Aufnahme verschmutzter medizinischer Geräte und eine zweite Öffnung (132, 232, 332) zum Spenden medizinischer Geräte besitzt;
eine Spendekammer (120, 220, 310'), konfiguriert, um im Wesentlichen innerhalb des Inneren der Einhausung (130, 230, 330) umschlossen zu sein und von dem Inneren der Einhausung (130, 230, 330) entfernt zu werden, wobei die Spendekammer (120, 220, 310') geeignet ist, medizinische Geräte zu enthalten und eine Zugangsöffnung (129, 223, 317) an einem unteren Ende der Spendekammer (120, 220, 310') zum Passieren von medizinischen Geräten aus der Spendekammer (120, 220, 310') besitzt, wobei die Zugangsöffnung (129, 223, 317) zum Fluchten mit der zweiten Öffnung (132, 232, 332) der Einhausung (130 230, 330) positioniert ist, um Passieren von medizinischen Geräten aus der Einhausung (130, 230, 330) zu erleichtern; und
eine Entsorgungskammer (110, 210, 310), konfiguriert, um im Wesentlichen innerhalb des Inneren der Einhausung (130, 230, 330) angrenzend an die Spendekammer (120, 220, 310') zu sein und von dem Inneren der Einhausung (130, 230, 330) entfernt zu werden, wobei die Entsorgungskammer (110, 210, 310) geeignet ist, verschmutzte medizinische Geräte zu sammeln, wobei die Entsorgungskammer (110, 210, 310) eine Einlassöffnung (111, 211, 317) an einem oberen Ende der Entsorgungskammer (110, 210, 310) zum Passieren von verschmutzten medizinischen Geräten in die Entsorgungskammer (110, 210, 310) besitzt, wobei die Einlassöffnung (111, 211, 317) zum Fluchten mit der ersten Öffnung (131, 231, 331) der Einhausung (130, 230, 330) positioniert ist, um Passieren von verschmutzten medizinischen Geräten in die Einhausung (130, 230, 330) zu erleichtern.

2. System zum Spenden und Entsorgen (200) medizinischer Geräte nach Anspruch 1, bei welchem die Spendekammer (220) und die Entsorgungskammer (210) aus einem einzigen, vereinheitlichten Körper gebildet sind.

3. System zum Spenden und Entsorgen (200) medizinischer Geräte nach Anspruch 1, zudem beinhaltend einen inneren Wandabschnitt (221), welcher die Spendekammer (220) und die Entsorgungskammer (210) trennt.

4. System zum Spenden und Entsorgen (200) medizinischer Geräte nach Anspruch 2, zudem beinhaltend einen Deckel (222), welcher die Spendekammer (220) und die Entsorgungskammer (210) abdeckt.

5. System zum Spenden und Entsorgen (200) medizinischer Geräte nach Anspruch 4, bei welchem der Deckel eine Tür (212) beinhaltet, welche proximal zur Einlassöffnung (211) der Entsorgungskammer (210) positioniert ist, wobei die Tür (212) eine offene Position besitzt, welche konfiguriert ist, um Zugang zu einem Inneren der Entsorgungskammer (210) zu bieten, und eine geschlossene Position, konfiguriert zum Verhindern des Zugangs zum Inneren der Entsorgungskammer (210).

6. System zum Spenden und Entsorgen (200) medizinischer Geräte nach Anspruch 5, wobei die Tür (212) zum Drehen zwischen der offenen und der geschlossenen Position montiert ist.

7. System zum Spenden und Entsorgen (100, 300) medizinischer Geräte nach Anspruch 1, bei welchem die Spendekammer (120, 310') und die Entsorgungskammer (110, 310) durch separate Behälter definiert sind.

8. System zum Spenden und Entsorgen (100, 300) medizinischer Geräte nach Anspruch 7, bei welchem die Spendekammer (120, 310') eine Einlassöffnung (129, 317) und einen Deckel (122, 315) beinhaltet, welcher beweglich in eine offene Position ist, in welcher medizinische Geräte in der Entsorgungskammer (120, 310') durch die Einlassöffnung (129, 317) abgelegt werden können.

9. System zum Spenden und Entsorgen (100, 300) medizinischer Geräte nach Anspruch 8, bei welchem der Deckel (122, 315) mit Scharnier(en) angrenzend an die Einlassöffnung (129, 317) verbunden ist, wobei der Deckel (122, 315) konfiguriert ist, um zwischen der offenen Position und einer geschlossenen Position zu schwenken.

10. System zum Spenden und Entsorgen (100) medizinischer Geräte nach Anspruch 7, bei welchem die Spendekammer (120) einen Durchgangsabschnitt (123) beinhaltet, welcher proximal zu einer Basis der Spendekammer (120) positioniert ist, wobei der Durchgangsabschnitt (123) mindestens teilweise die Zugangsöffnung (129) definiert.

11. System zum Spenden und Entsorgen (100) medizinischer Geräte nach Anspruch 10, bei welchem der Durchgangsabschnitt (123) eine Tür beinhaltet, welche zwischen einer offenen Position und einer geschlossenen Position beweglich ist, und bei welchem der Durchgangsabschnitt (123) einen Durchgang durch die Zugangsöffnung (129) bereitstellt, wenn die Tür in der offenen Position ist und der Durchgangsabschnitt (123) mindestens teilweise die Zugangsöffnung (129) versperrt, wenn die Tür in der geschlossenen Position ist.

12. System zum Spenden und Entsorgen (100) medizinischer Geräte nach Anspruch 11, bei welchem die Tür zwischen der offenen Position und der geschlossenen Position schwenkbar ist.

13. System zum Spenden und Entsorgen (100) medizinischer Geräte nach Anspruch 11, bei welchem die Tür eine Wand besitzt, welche mindestens teilweise den Durchgangsabschnitt (123) bildet, wenn sie in der offenen Position ist.

14. System zum Spenden und Entsorgen (100) medizinischer Geräte nach Anspruch 13, wobei die Tür Seitenwandabschnitte und einen Vorderwandabschnitt besitzt, welcher sich zwischen den Seitenwandabschnitten erstreckt.

15. System zum Spenden und Entsorgen (100, 200, 300) medizinischer Geräte nach Anspruch 1, bei welchem die Spendekammer (120, 220, 310') mindestens ein medizinisches Gerät enthält.

16. System zum Spenden und Entsorgen (100, 200, 300) medizinischer Geräte nach Anspruch 15,
bei welchem mindestens ein medizinisches Gerät aus der Gruppe gewählt ist, bestehend aus Spritzen, scharfen Instrumenten, Zungenspateln oder Abstrichstäbchen.

17. System zum Spenden und Entsorgen (100, 300) medizinischer Geräte nach Anspruch 7, bei welchem die Spendekammer (110, 310) eine Tür (112, 370) beinhaltet, welche proximal zur Einlassöffnung (111, 317) positioniert ist, wobei die Tür (112, 370) eine offene Position besitzt, welche konfiguriert ist, um Zugang zu einem Inneren der Entsorgungskammer (110, 310) zu bieten, und eine geschlossene Position, konfiguriert zum Verhindern des Zugangs zum Inneren der Entsorgungskammer (110, 310).

18. System zum Spenden und Entsorgen (100, 300) medizinischer Geräte nach Anspruch 17,
wobei die Tür (112, 370) zum Drehen zwischen der offenen und der geschlossenen Position montiert ist.

19. System zum Spenden und Entsorgen (300) medizinischer Geräte nach Anspruch 1, bei welchem die Spendekammer (310') und die Entsorgungskammer (310) jeweils durch im Wesentlichen identische Behälter (310, 310') definiert sind.

20. System zum Spenden und Entsorgen (300) medizinischer Geräte nach Anspruch 19, bei welchem die Behälter (310, 310'), welche die Spendekammer (310') und die Entsorgungskammer (310) definieren, jeweils einen Deckel (315) besitzen.

## Revendications

1. Système de délivrance et de mise au rebut d'instruments médicaux (100, 200, 300) comprenant :
une enceinte (130, 230, 330) qui définit un intérieur et qui comporte une première ouverture (131, 231, 331) pour recevoir des instruments médicaux souillés et une seconde ouverture (132, 232, 332) pour délivrer des instruments médicaux ;
une chambre de délivrance (120, 220, 310') qui est configurée de manière à ce qu'elle soit sensiblement renfermée à l'intérieur de l'intérieur de l'enceinte (130, 230, 330) et à ce qu'elle soit enlevée hors de l'intérieur de l'enceinte (130, 230, 330), ladite chambre de délivrance (120, 220, 310') étant adaptée de manière à ce qu'elle contienne des instruments médicaux et comportant une ouverture d'accès (129, 223, 317) au niveau d'une extrémité inférieure de la chambre de délivrance (120, 220, 310') pour le passage d'instruments médicaux depuis ladite chambre de délivrance (120, 220, 310'), ladite ouverture d'accès (129, 223, 317) étant positionnée de sorte qu'elle soit alignée avec la seconde ouverture (132, 232, 332) de l'enceinte (130, 230, 330) afin de faciliter le passage d'instruments médicaux depuis l'enceinte (130, 230, 330) ; et
une chambre de mise au rebut (110, 210, 310) qui est configurée de manière à ce qu'elle soit de façon substantielle à l'intérieur de l'intérieur de l'enceinte (130, 230, 330) de sorte qu'elle soit adjacente à ladite chambre de délivrance (120, 220, 310') et de manière à ce qu'elle soit enlevée hors de l'intérieur de l'enceinte (130, 230, 330), ladite chambre de mise au rebut (110, 210, 310) étant adaptée de manière à ce qu'elle collecte des instruments médicaux souillés, ladite chambre de mise au rebut (110, 210, 310) comportant une ouverture d'entrée (111, 211, 317) au niveau d'une extrémité supérieure de la chambre de mise au rebut (110, 210, 310) pour le passage d'instruments médicaux souillés jusqu'à l'intérieur de ladite chambre de mise au rebut (110, 210, 310), ladite ouverture d'entrée (111, 211, 317) étant positionnée de sorte qu'elle soit alignée avec la première ouverture (131, 231, 331) de l'enceinte (130, 230, 330) afin de faciliter le passage d'instruments médicaux souillés jusqu'à l'intérieur de l'enceinte (130, 230, 330).

2. Système de délivrance et de mise au rebut d'instruments médicaux (200) selon la revendication 1, ladite chambre de délivrance (220) et ladite chambre de mise au rebut (210) étant formées à partir d'un unique corps unifié.

3. Système de délivrance et de mise au rebut d'instruments médicaux (200) selon la revendication 1, comprenant en outre une partie de paroi intérieure (221) qui sépare ladite chambre de délivrance (220) et ladite chambre de mise au rebut (210).

4. Système de délivrance et de mise au rebut d'instruments médicaux (200) selon la revendication 2, comprenant en outre un couvercle (222) qui recouvre ladite chambre de délivrance (220) et ladite chambre de mise au rebut (210).

5. Système de délivrance et de mise au rebut d'instruments médicaux (200) selon la revendication 4, ledit couvercle incluant une porte (212) qui est positionnée de façon proximale par rapport à ladite ouverture d'entrée (211) de ladite chambre de mise au rebut (210), ladite porte (212) présentant une position ouverte qui est configurée de manière à ce qu'elle permette un accès à un intérieur de ladite chambre de mise au rebut (210) et une position fermée qui est configurée de manière à ce qu'elle empêche un accès à l'intérieur de ladite chambre de mise au rebut (210).

6. Système de délivrance et de mise au rebut d'instruments médicaux (200) selon la revendication 5, ladite porte (212) étant montée à rotation entre lesdites positions ouverte et fermée.

7. Système de délivrance et de mise au rebut d'instruments médicaux (100, 300) selon la revendication 1, dans lequel ladite chambre de délivrance (120, 310') et ladite chambre de mise au rebut (110, 310) sont définies par des conteneurs séparés.

8. Système de délivrance et de mise au rebut d'instruments médicaux (100, 300) selon la revendication 7, ladite chambre de délivrance (120, 310') incluant une ouverture d'entrée (129, 317) et un couvercle (122, 315) qui peut être déplacé jusqu'à une position ouverte dans laquelle des instruments médicaux peuvent être déposés à l'intérieur de ladite chambre de délivrance (120, 310') au travers de ladite ouverture d'entrée (129, 317).

9. Système de délivrance et de mise au rebut d'instruments médicaux (100, 300) selon la revendication 8, ledit couvercle (122, 315) étant connecté par charnière de sorte qu'il soit adjacent à ladite ouverture d'entrée (129, 317), ledit couvercle (122, 315) étant configuré de manière à ce qu'il pivote entre ladite position ouverte et une position fermée.

10. Système de délivrance et de mise au rebut d'instruments médicaux (100) selon la revendication 7, ladite chambre de délivrance (120) incluant une partie de bac (123) qui est positionnée de façon proximale par rapport à une base de ladite chambre de délivrance (120), ladite partie de bac (123) définissant au moins partiellement ladite ouverture d'accès (129).

11. Système de délivrance et de mise au rebut d'instruments médicaux (100) selon la revendication 10, dans lequel ladite partie de bac (123) comprend une porte qui peut être déplacée entre une position ouverte et une position fermée, et dans lequel ladite partie de bac (123) assure un passage au travers de ladite ouverture d'accès (129) lorsque ladite porte est dans ladite position ouverte, et ladite partie de bac (123) obstrue au moins partiellement ladite ouverture d'accès (129) lorsque ladite porte est dans ladite position fermée.

12. Système de délivrance et de mise au rebut d'instruments médicaux (100) selon la revendication 11, dans laquelle ladite porte peut pivoter entre ladite position ouverte et ladite position fermée.

13. Système de délivrance et de mise au rebut d'instruments médicaux (100) selon la revendication 11, ladite porte comportant une paroi qui forme au moins partiellement ladite partie de bac (123) lorsqu'elle est dans ladite position ouverte.

14. Système de délivrance et de mise au rebut d'instruments médicaux (100) selon la revendication 13, ladite porte comportant des parties de paroi latérale et une partie de paroi avant qui s'étend entre lesdites parties de paroi latérale.

15. Système de délivrance et de mise au rebut d'instruments médicaux (100, 200, 300) selon la revendication 1,
ladite chambre de délivrance (120, 220, 310') contenant au moins un instrument médical.

16. Système de délivrance et de mise au rebut d'instruments médicaux (100, 200, 300) selon la revendication 15,
dans lequel ledit au moins un instrument médical est sélectionné parmi le groupe constitué par les seringues, les instruments médicaux pointus, les abaisse-langue et les porte-coton.

17. Système de délivrance et de mise au rebut d'instruments médicaux (100, 300) selon la revendication 7,
ladite chambre de mise au rebut (110, 310) incluant une porte (112, 370) qui est positionnée de façon proximale par rapport à ladite ouverture d'entrée (111, 317), ladite porte (112, 370) présentant une position ouverte qui est configurée de manière à ce qu'elle permette un accès à un intérieur de ladite chambre de mise au rebut (110, 310) et une position fermée qui est configurée de manière à ce qu'elle empêche un accès à l'intérieur de ladite chambre de mise au rebut (110, 310).

18. Système de délivrance et de mise au rebut d'instruments médicaux (100, 300) selon la revendication 17,
ladite porte (112, 370) étant montée à rotation entre lesdites positions ouverte et fermée.

19. Système de délivrance et de mise au rebut d'instruments médicaux (300) selon la revendication 1, dans lequel ladite chambre de délivrance (310') et ladite chambre de mise au rebut (310) sont chacune définies par des conteneurs sensiblement identiques (310,310').

20. Système de délivrance et de mise au rebut d'instruments médicaux (300) selon la revendication 19,
lesdits conteneurs (310, 310') qui définissent ladite chambre de délivrance (310') et ladite chambre de mise au rebut (310) comportant chacun un couvercle (315).
